# EUROPEAN PATENT APPLICATION

(11) **EP 3 135 282 A1**
(43) Date of publication of application: **01.03.2017**
(21) Application number: 16188566.0
(22) Date of filing: 07.02.2008
(51) Int. Cl.: A61K 31/225, A61P 25/00

(54) **NEUROPROTECTION IN DEMYELINATING DISEASES**

(30) Priority: 08.02.2007 US 888925 P
(62) Divisional of application: 08737369.2
(71) Applicant: Biogen MA Inc., Cambridge, MA 02142 (US)
(72) Inventor: Ralf, Gold, 44799 Bochum (DE)
(74) Representative: Pohlman, Sandra M.

(57) **Abstract**

Methods of treating neurological disorders characterized by extensive demyelination and/or axonal loss are provided. Examples of such disorders include secondary progressive multiple sclerosis and Devic's disease. The disclosed methods include administering to a subject having such a disorder a therapeutically effective amount of, for example, dimethyl fumarate or monomethyl fumarate.

## Description

Provided are methods and compositions for treating demyelinating disorders and related types of disorders of the nervous system, including for example, multiple sclerosis, among other things.

Multiple sclerosis (MS) is an autoimmune disease with the autoimmune activity directed against central nervous system (CNS) antigens. The disease is characterized by inflammation in parts of the CNS, leading to the loss of the myelin sheathing around neuronal axons (demyelination), axonal loss, and the eventual death of neurons, oligodenrocytes and glial cells. For a comprehensive review of MS and current therapies, see, e.g., McAlpine's Multiple Sclerosis, by Alastair Compston et al., 4th edition, Churchill Livingstone Elsevier, 2006.

An estimated 2,500,000 people in the world suffer from MS. It is one of the most common diseases of the CNS in young adults. MS is a chronic, progressing, disabling disease, which generally strikes its victims some time after adolescence, with diagnosis generally made between 20 and 40 years of age, although onset may occur earlier. The disease is not directly hereditary, although genetic susceptibility plays a part in its development. MS is a complex disease with heterogeneous clinical, pathological and immunological phenotype.

There are four major clinical types of MS: 1) relapsing-remitting MS (RR-MS), characterized by clearly defined relapses with full recovery or with sequelae and residual deficit upon recovery; periods between disease relapses characterized by a lack of disease progression; 2) secondary progressive MS (SP-MS), characterized by initial relapsing remitting course followed by progression with or without occasional relapses, minor remissions, and plateaus; 3) primary progressive MS (PP-MS), characterized by disease progression from onset with occasional plateaus and temporary minor improvements allowed; and 4) progressive relapsing MS (PR-MS), characterized by progressive disease onset, with clear acute relapses, with or without full recovery; periods between relapses characterized by continuing progression.

Clinically, the illness most often presents as a relapsing-remitting disease and, to a lesser extent, as steady progression of neurological disability. Relapsing-remitting MS (RR-MS) presents in the form of recurrent attacks of focal or multifocal neurologic dysfunction. Attacks may occur, remit, and recur, seemingly randomly over many years. Remission is often incomplete and as one attack follows another, a stepwise downward progression ensues with increasing permanent neurological deficit. The usual course of RR-MS is characterized by repeated relapses associated, for the majority of patients, with the eventual onset of disease progression. The subsequent course of the disease is unpredictable, although most patients with a relapsing-remitting disease will eventually develop secondary progressive disease. In the relapsing-remitting phase, relapses alternate with periods of clinical inactivity and may or may not be marked by sequelae depending on the presence of neurological deficits between episodes. Periods between relapses during the relapsing-remitting phase are clinically stable. On the other hand, patients with progressive MS exhibit a steady increase in deficits, as defined above and either from onset or after a period of episodes, but this designation does not preclude the further occurrence of new relapses.

MS pathology is, in part, reflected by the formation of focal inflammatory demyelinating lesions in the white matter, which are the hallmarks in patients with acute and relapsing disease. In patients with progressive disease, the brain is affected in a more global sense, with diffuse but widespread (mainly axonal) damage in the normal appearing white matter and massive demyelination also in the grey matter, particularly, in the cortex.

Most current therapies for MS are aimed at the reduction of inflammation and suppression or modulation of the immune system. As of 2006, the available treatments for MS reduce inflammation and the number of new episodes but not all of the treatments have an effect on disease progression. A number of clinical trials have shown that the suppression of inflammation in chronic MS rarely significantly limits the accumulation of disability through sustained disease progression, suggesting that neuronal damage and inflammation are independent pathologies. Thus, in advanced stages of MS, neurodegeneration appears to progress even in the absence of significant inflammation. Therefore, slowing demyelination, or promoting CNS remyelination as a repair mechanism, or otherwise preventing axonal loss and neuronal death are some of the important goals for the treatment of MS, especially, in the case of progressive forms of MS such as SP-MS.

Fumaric acid esters, such as dimethyl fumarate (DMF), have been previously proposed for the treatment of MS (see, e.g., Schimrigk et al., Eur. J. Neurol., 2006, 13(6):604-10; Drugs R&D, 2005, 6(4):229-30; U.S. Patent No. 6,436,992).

DMF and monomethyl fumarate (MMF) can exert neuroprotective effects such as reduction in demyelination and axonal damage in a mouse MS model with characteristic features of advanced stages of chronic forms of MS. Although many well characterized rodent and primate models for MS exist, only recently have the characteristic features of progressive MS been identified in select animal models. Under the conditions tested, the neuroprotective effects of DMF and MMF appeared to be independent of their effect, if any, on inflammation, suggesting that use of these compounds may be advantageous in treating pathologies that exhibit progressive neurodegeneration even in the absence of a substantial inflammatory component.

Provided are methods of treating neurological disorders characterized by extensive demyelination and/or axonal loss such as, for example, is present in a patient with a score of 3 or higher on the Expanded Disability Status Scale (EDSS) or in a patient who has more than 10 hypointense T1 lesions.

In some embodiments, the subject has a progressive form of a demyelinating disorder, e.g., MS (e.g., primary progressive or secondary progressive MS) and Devic's disease. In some cases, as for example, in secondary progressive MS, the disorder may be further characterized by initial inflammation followed by progressive demyelination and/or axonal loss.

The disease progression in the subject can be such that the subject exhibits at least a 1-point increase in the EDSS score in the previous year and/or at least a 25% increase in T1 lesion load over the previous year.

In some embodiments, the methods comprise administering to the subject having the neurological disorder a therapeutically effective amount of at least one compound of Formula I: 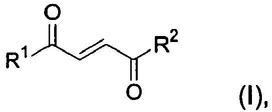 wherein R¹ and R² are independently selected from OH, O⁻, and (C₁₋₆)alkoxy, or a pharmaceutically acceptable salt thereof. In nonlimiting illustrative embodiments, the compound is dimethyl fumarate (R¹ is CH₃ and R² is CH₃) or monomethyl fumarate (R¹ is CH₃ and R² is O⁻ or OH, e.g., a pharmaceutically acceptable salt of monomethyl fumarate, e.g., specifically, Ca-MMF).

In some embodiments, the compound is administered in an amount and for a period of time sufficient to reduce demyelination and/or axonal death in the subject. In some embodiments, the compound is administered in an amount and for a period of time sufficient to slow the accumulation of disability in the subject.

Some embodiments provide methods in which a pharmaceutical preparation that contains one or both of DMF and MMF, may be administered orally to a subject with secondary progressive MS or another demyelinating disease described below.

Other features and embodiments will be apparent from the following description and the claims.

### BRIEF DESCRIPTION OF THE FIGURES

**Figure 1** shows the clinical course of active myelin oligodendrocyte protein-induced experimental autoimmune encephalomyelitis (MOG-EAE) in DMF-treated, MMF-treated or methocel-fed control mice. Animals were pooled from two experiments (total number of 14 mice per group). Mice were followed until the late phase of the disease (72 days post-immunization (p.i.)). At that time point, DMF-treated mice exhibited a significantly milder disease course.
**Figure 2A** is a bar graph showing the average level of demyelination (% white matter) in a mouse MOG-EAE model 72 days p.i., following administration of DMF, MMF, and methocel (as a control). The results show that the level of demyelination was reduced in mice treated with DMF and MMF.
**Figure 2B** is a bar graph showing the level of relative axonal density in a mouse MOG-EAE model 72 days p.i., following administration of DMF, MMF, and methocel (as a control). The results show that the level of axonal loss was reduced in mice treated with DMF and MMF.
**Figure 3A** shows results of a blinded histological analysis of CD 3 positive T cells infiltrating the spinal cord 72 days after induction of MOG-EAE. Numbers of infiltrating T cells were not significantly different between MMF-treated, DMF-treated, and methocel-fed control mice.
**Figure 3B** shows results of a blinded histological analysis of Mac-3 positive macrophages and microglia infiltrating the spinal cord 72 days after induction of MOG-EAE. Numbers of infiltrating macrophages and microglia were not significantly different between MMF-treated, DMF-treated, and methocel-fed control mice.

Certain terms are defined in this section; additional definitions are provided throughout the description.

The terms "disease" and "disorder" are used interchangeably herein.

The term "neurological disorder" refers to disorders of the nervous system that result in impairment of neuronal mediated functions and includes disorders of the central nervous system (e.g., the brain, spinal cord) as well as the peripheral nervous system.

The term "neuroprotection" refers to prevention or a slowing in neuronal degeneration, including, for example, demyelination and/or axonal loss, and optionally, neuronal and oligodendrocyte death.

The terms "therapeutically effective dose" and "therapeutically effective amount" refer to that amount of a compound which results in prevention or delay of onset or amelioration of symptoms of a neurological disorder in a subject or an attainment of a desired biological outcome, such as reduced neurodegeneration (e.g., demyelination, axonal loss, or neuronal death) or slowing in the accumulation of physical disability (e.g., as indicated by, e.g., a reduced rate of worsening of a clinical score (e.g., EDSS) or another suitable parameter indicating disease state (e.g., the number of T1 lesions, reduced number of Gd+ lesions, etc.)).

The term "treating" refers to administering a therapy in an amount, manner, and/or mode effective to improve a condition, symptom, or parameter associated with a disorder or to prevent progression of a disorder, to either a statistically significant degree or to a degree detectable to one skilled in the art. An effective amount, manner, or mode can vary depending on the subject and may be tailored to the subject. For neurological disorders referred herein, the treatments offered by the methods disclosed herein aim at improving the conditions (or lessening the detrimental effects) of the disorders and not necessarily at completely eliminating or curing the disorders.

Unless otherwise specified, the term "MMF" refers to monomethyl fumarate in the form of acid (methyl hydrogen fumarate, also known as "MHF") as well as to its corresponding salts.

In some embodiments, the methods comprise administering to a subject having the neurological disorder a therapeutically effective amount of at least one compound of Formula I: 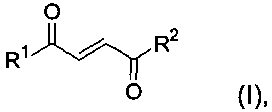 wherein R¹ and R² are independently selected from OH, O⁻, and (C₁₋₆)alkoxy, or a pharmaceutically acceptable salt thereof. (C₁₋₆)alkoxy can be chosen from, for example, (C₁₋₅)alkoxy, (C₁₋₄)alkoxy, (C₁₋₃)alkoxy, ethoxy, methoxy, (C₂₋₃)alkoxy, (C₂₋₄)alkoxy, (C₂₋₅)alkoxy, and (C₁₋₆)alkoxy. In some embodiments of the compounds of Formula I, the pharmaceutically acceptable salt is a salt of a metal (M) cation, wherein M can be an alkali, alkaline earth, or transition metal such as Li, Na, K, Ca, Zn, Sr, Mg, Fe, or Mn.
In nonlimiting illustrative embodiments, the compound of Formula I is dimethyl fumarate (R¹ is CH₃ and R² is CH₃) or monomethyl fumarate (R¹ is CH₃ and R² is O⁻ or OH, e.g., a pharmaceutically acceptable salt of monomethyl fumarate, e.g., specifically, Ca-MMF).

Also provided are methods of treating a patient having a neurological disorder characterized by extensive demyelination and/or axonal loss. For example, the degree of demyelination and/or axonal loss may be such as present in a patient with a score of 3, 3.5, 4, 4.5, 5, 5.5, 6, 6.5, 7 or higher on the Expanded Disability Status Scale (EDSS; see Table 1 below). Other suitable measurement scales can be also used (see, e.g., pp. 288-291 in McAlpine's Multiple Sclerosis, by Alastair Compston et al., 4th edition, Churchill Livingstone Elsevier, 2006).

**Table 1. Expanded Disability Status Scale (EDSS)**

| | |
|---|---|
| **0** | Normal neurological examination (all grade 0 in functional systems [FS]; cerebral grade 1 acceptable) |
| **1** | No disability, minimal signs in 1 FS (i.e. grade 1 excluding cerebral grade 1) |
| **1.5** | No disability, minimal signs in >1 FS (>1 grade 1 excluding cerebral grade 1) |
| **2** | Minimal disability in 1 FS (1 FS grade 2, others 0 or 1) |
| **2.5** | Minimal disability in 2 FS (2 FS grade 2, others 0 or 1) |
| **3** | Moderate disability in 1 FS (1 FS grade 3, others 0 or 1), or mild disability in 3-4 FS (3-4 FS grade 2, others 0 or 1) though fully ambulatory |
| **3.5** | Fully ambulatory but with moderate disability in 1 FS (1 FS grade 3) and 1-2 FS grade 2; or 2 FS grade 3; or 5 FS grade 2 (others 0 or 1) |
| **4** | Fully ambulatory without aid, self-sufficient, up and about some 12 hours a day despite relatively severe disability consisting of 1 FS grade 4 (others 0 or 1), or combinations of lesser grades exceeding limits of previous steps. Able to walk without aid or rest some 500 m |
| **4.5** | Fully ambulatory without aid, up and about much of the day, able to work a full day, may otherwise have some limitation of full activity or require minimal assistance; characterized by relatively severe disability, usually consisting of 1 FS grade 4 (others 0 or 1) or combinations of lesser grades exceeding limits of previous steps. Able to walk without aid or rest for some 300 m |
| **5** | Ambulatory without aid or rest for about 200 m; disability severe enough to impair full daily activities (e.g. to work full day without special provisions). (Usual FS equivalents are 1 grade 5 alone, others 0 or 1; or combination of lesser grades usually exceeding specifications for step 4.0) |
| **5.5** | Ambulatory without aid or rest for about 100 m, disability severe enough to preclude full daily activities. (Usual FS equivalents are 1 grade 5 alone, others 0 or 1; or combination of lesser grades usually exceeding those for step 4.0) |
| **6** | Intermittent or unilateral constant assistance (cane, crutch or brace) required to walk about 100 m with or without resting. (Usual FS equivalents are combinations with >2 FS grade 3+) |
| **6.5** | Constant bilateral assistance (canes, crutches or braces) required to walk about 20 m without resting. (Usual FS equivalents are combinations with >2 FS grade 3+) |
| **7** | Unable to walk beyond about 5 m even with aid, essentially restricted to wheelchair; wheels self in standard wheelchair and transfers alone; up and about in wheelchair some 12 hours a day. (Usual FS equivalents are combinations with >1 FS grade 4+; very rarely, pyramidal grade 5 alone) |
| **7.5** | Unable to take more than a few steps; restricted to wheelchair, may need aid in transfer; wheels self but cannot carry on in standard wheelchair a full day; may require motorized wheelchair. (Usual FS equivalents are combinations with >1 FS grade 4+) |
| **8** | Essentially restricted to bed or chair or perambulated in wheelchair, but may be out of bed itself much of the day; retains many self-care functions; generally has effective use of arms. (Usual FS equivalents are combinations, generally 4+ in several systems) |
| **8.5** | Essentially restricted to bed much of the day; has some effective use of arm(s); retains some self-care functions. (Usual FS equivalents are combinations, generally 4+ in several systems) |
| **9** | Helpless bedridden patient; can communicate and eat. (Usual FS equivalents are combinations, mostly grade 4+) |
| **9.5** | Totally helpless bedridden patient; unable to communicate effectively or eat/swallow. (Usual FS equivalents are combinations, almost all grade 4+) |
| **10** | Death due to multiple sclerosis |

As another example, the degree of demyelination and/or axonal loss may be such as that in a patient who has more than 10, 12, 15, 20 or more hypointense T1 lesions. The number of such lesions can be determined, for example, by routine MRI methods.

In some embodiments, the subject has a progressive form of a demyelinating disorder, e.g., MS (e.g., primary progressive or secondary progressive MS) and Devic's disease. In some cases, as for example, in secondary progressive MS, the subject may have a disorder that may be characterized by initial inflammation followed by progressive demyelination and/or axonal loss. The diagnosis of MS may be performed as per McDonald's criteria as described in, e.g., McDonald et al., Ann. Neurol., 2001, 50:120-127; or the 2005 revised criteria as described in, e.g., Polman et al., Annals of Neurology, 2005, 58(6):840-846.

In some embodiments, the subject being treated has secondary progressive MS and an EDSS score of more than 5, 5.5, 6, 6.5, 7, or higher.

The disease progression in the subject can be such that the subject exhibits at least a 1-, 1.5-, 2-, 2.5-, 3-, 3.5-point or greater increase in the EDSS score in the previous year and/or at least a 25%, 30%, 40%, 50%, 75%, or 100% increase in T1 lesion load over the previous year.

Additional parameters describing the subjects with an advanced stage demyelinating disorder can be (a) T2 lesion volume more than 15 cm³ and/or (b) corpus callosum area less than 400 mm².

Examples of other demyelinating neurological disorders suitable for treatment by the methods disclosed include optic neuritis, acute inflammatory demyelinating polyneuropathy (AIDP), chronic inflammatory demyelinating polyneuropathy (CIDP), acute transverse myelitis, progressive multifocal leucoencephalopathy (PML), acute disseminated encephalomyelitis (ADEM) or other hereditary disorders (e.g., leukodystrophies, Leber's optic atrophy, and Charcot-Marie-Tooth disease).

AIDP, for example, is an acute or subacute monophasic peripheral nerve disorder. Patients generally experience proximal, distal or generalized weakness. Over half of the patients with AIDP have a prior infection within the past two weeks, and the neurological symptoms rapidly progress over the next few days or weeks, reach a plateau for a few more weeks, and then eventually improve over months. Diagnosis can be made by a combination of history and physical examination, nerve conduction analysis, EMG, and CSF analysis.

As another example, progressive multifocal leukoencephalopathy (PML) is a demyelinating disorder caused by a polyoma virus (the JC virus). It rarely affects immunocompetent people even though two-thirds of the population has been exposed to the JC virus. The JC virus often attacks oligodendrocytes, thereby causing demyelination. Most of the patients affected by PML are immunosuppressed, e.g., transplant recipients, lymphoma or AIDS patients. PML is generally progressive and frequently multifocal. The demyelinating lesions, which can be monitored by CT and MRI scans, often contain breakdown products of myelin within foamy macrophages. Astrocytes can be observed with atypical pleomorphic nuclei, and viral inclusions observed in enlarged oligodendroglial nuclei. Because PML patients are predominately already immunosuppressed, a treatment for demyelination and/or axonal in PML that does not further compromise the immune system may be advantageous (e.g., as in accordance with some embodiments of the methods disclosed herein).

In certain embodiments, the methods provide treated subjects neuroprotective effects, e.g., protection of the neuronal cells or nerve processes (axons) from death or being damaged. These neuroprotective effects do not necessarily eliminate all of the damages or degeneration, but rather, delay or even halt the progress of the degeneration or a prevention of the initiation of the degeneration process or an improvement to the pathology of the disorder. In some embodiments the methods offer neuroprotection to at least one part of the nervous system, such as for example the central nervous system, e.g., hippocampus, cerebellum, spinal cord, cortex (e.g., motor or somatosensory cortex), striatum, basal forebrain (cholenergic neurons), ventral mesencephalon (cells of the substantia nigra), and the locus ceruleus (neuroadrenaline cells of the central nervous system).

In some embodiments of the methods the subject being treated is a subject in need of neuroprotection, including subjects who have extensive demyelination and axonal loss such as subjects that have secondary progressive MS or another demyelinating disorder as specified above. In some embodiments of the methods the subjects are mammalian, e.g., rodents or another laboratory animal, e.g., a non-human primate. In some embodiments, the subject is human. In some embodiments, the human subject is older than 55, 57, 60, 65, or 70 years of age.

In some embodiments, the compound is administered in an amount and for a period of time sufficient to reduce demyelination and/or axonal death in the subject.

In some embodiments, the compound is administered in an amount and for a period of time sufficient to slow the accumulation of disability, e.g., progression in disability, in the subject. Accumulation of disability/progression in disability is reflected by, for example, an increase in the EDSS score and may be measured as the length of time to an increase of at least 1 point in the EDSS score. For example, the compound may be administered in an amount and for a period of time sufficient to sustain an increase in the EDSS score within 1 point or less for 3, 4, 5, 6, 7, 8, 9, 10, 11, 12, 24, 36 months or longer.

In some embodiments the method includes treating the subject with a therapeutically effective amount of at least one compound chosen from DMF and MMF. For DMF or MMF, the therapeutically effective amount can range from about 1 mg/kg to about 50 mg/kg (e.g., from about 2.5 mg/kg to about 20 mg/kg or from about 2.5 mg/kg to about 15 mg/kg). Effective doses will also vary, as recognized by those skilled in the art, dependent on route of administration, excipient usage, and the possibility of co-usage with other therapeutic treatments including use of other therapeutic agents. For example, an effective dose of DMF or MMF to be administered to a subject, for example orally, can be from about 0.1 g to about 1 g per day, for example, from about 200 mg to about 800 mg per day (e.g., from about 240 mg to about 720 mg per day; or from about 480 mg to about 720 mg per day; or about 720 mg per day). For example, 720 mg per day may be administered in separate administrations of 2, 3, 4, or 6 equal doses.

The therapeutic compound (e.g., DMF or MMF) can be administered by any method that permits the delivery of the compound for treatment of neurological disorders. For instance, the therapeutic compound can be administered via pills, tablets, microtablets, pellets, micropellets, capsules (e.g., containing microtablets), suppositories, liquid formulations for oral administration, and in the form of dietary supplements. The pharmaceutically acceptable compositions can include well-known pharmaceutically acceptable excipients, e.g., if the composition is an aqueous solution containing the active agent, it can be an isotonic saline, 5% glucose, or others. Solubilizing agents such as cyclodextrins, or other solubilizing agents well known to those familiar with the art, can be utilized as pharmaceutical excipients for delivery of the therapeutic compound. See, e.g., US Patent Nos. 6,509,376 and 6,436,992 for some formulations containing DMF and/or MMF. As to route of administration, the compositions can be administered orally, intranasally, transdermally, subcutaneously, intradermally, vaginally, intraaurally, intraocularly, intramuscularly, buccally, rectally, transmucosally, or via inhalation, or intravenous administration. In some embodiments DMF or MMF is administered orally.

In some embodiments, the method comprises administering orally a capsule containing a pharmaceutical preparation consisting essentially of 60-240 mg (e.g., 120 mg) of dimethyl fumarate in the form of enteric-coated microtablets. In some embodiments, the mean diameter of such microtablets is 1-5 mm, e.g., 1-3 mm or 2 mm.

The therapeutic compound can be administered in the form of a sustained or controlled release pharmaceutical formulation. Such formulation can be prepared by various technologies by a skilled person in the art. For example, the formulation can contain the therapeutic compound, a rate-controlling polymer (i.e., a material controlling the rate at which the therapeutic compound is released from the dosage form) and optionally other excipients. Some examples of rate-controlling polymers are hydroxy alkyl cellulose, hydroxypropyl alkyl cellulose (e.g., hydroxypropyl methyl cellulose, hydroxypropyl ethyl cellulose, hydroxypropyl isopropyl cellulose, hydroxypropyl butyl cellulose and hydroxypropyl hexyl cellulose), poly(ethylene)oxide, alkyl cellulose (e.g., ethyl cellulose and methyl cellulose), carboxymethyl cellulose, hydrophilic cellulose derivatives, and polyethylene glycol, compositions described in WO 2006/037342.

The following example is illustrative and does not limit the scope of the disclosure or the claims.

### Example

*Treatment conditions*-Severe, chronic EAE was actively induced in C57BL/6 mice (form Harlan, Borchen, Germany) using 50 µg of the encephalitogenic peptide MOG 35-55 (purchased from Charite, Berlin, Germany, see also Mendel et al. (1995) Eur. J. Immunol., 25:1951-1959) and pertussis toxin (2 x 400 ng), essentially as described in Malipiero et al. (1997) Eur. J. Immunol., 27:3151-3160. Treatment started at day -20 before the injection of MOG. The following compounds were administered orally to three groups of mice as follows: 1) Ca-monomethyl fumarate 5 mg/kg body weight bid; 2) dimethyl fumarate 15 mg/kg body weight bid; 3) 0.08% methocel as control. For analyzing the clinical course, data was pooled from two experiments (one experiment with 6 mice and another with 8 mice per group yielding a total number of 14 mice per experimental group).

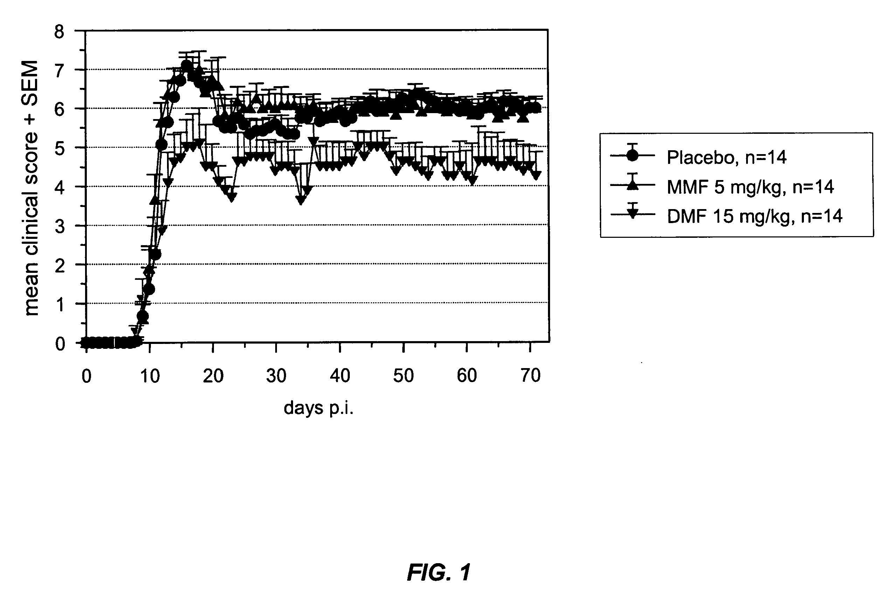

*Clinical evaluation*-Symptoms were scored 1-10 on a daily basis as described in Linker et al., Nat. Med., 2002, 29:626-632 (see also Hartung et al., Brain, 1988, 11, 1039-1059). Briefly, disease severity was scored as follows: 0, normal; 1, reduced tone of tail; 2, limp tail, impaired righting; 3, absent righting; 4, gait ataxia; 5, mild paraparesis of hindlimbs; 6, moderate paraparesis; 7, severe paraparesis or paraplegia; 8, tetraparesis; 9, moribund; 10, death. Relapses were defined as deterioration by 2 points or more within 2 days. Figure 1 shows the clinical course of active MOG-EAE in DMF-treated, MMF-treated or methocel-fed control mice. Animals were pooled from two experiments (total number of 14 mice per group). Mice were followed until the late phase of the disease (72 days p.i.). At that time point, DMF treated mice exhibited a significantly milder disease course. 15 mg/kg DMF was effective to reduce the clinical score up to 72 days p.i., whereas 5 mg/kg MMF was not sufficient to significantly affect the clinical score under the tested conditions. Although at the tested dose, 5 mg/kg, MMF did not have an effect on the clinical score, it did show a significant positive effect based on the histological examination (see below; reduced demyelination and axonal loss.)

*Histology*-One experiment was terminated on day 72 p.i. for histologic evaluation. At that time point, 6 mice in the MMF and 6 mice in the control group were available for analysis. The DMF group consisted of 4 mice (2 non-EAE related drop-outs). Mice were anesthetized with ether, bled and perfused with 25 ml Ringer solution and 10 ml of 4% paraformaldehyde in buffered PBS. Spinal cord was dissected out and fixed overnight in 4% paraformaldehyde in buffered PBS at 4°C before embedding in paraffin. Paraffin sections were stained with hematoxylin and eosin for visualization of inflammatory infiltrates and Luxol fast blue for visualization of demyelination. Coded sections from cervical, thoracic and lumbar spinal cord were evaluated by a blinded observer by means of overlaying a stereological grid and counting mean CD3 and Mac-3 positive cells within 3 visual fields (each 0.096 mm²) with the most intense pathology under a 400-fold magnification. The extent of demyelination was assessed by relating the number of grid squares with demyelination to the total number of grid squares containing white matter over an average of 8-10 independent levels of spinal cord per mouse. CD3, Mac-3 positive cells and APP positive axons were quantified on 3 representative sections, each one of cervical, thoracic and lumbar spinal cord by counting 2 defined areas with the most intense pathology under a 40-fold magnification. Histological evaluation was performed as described in Eugster et al., Eur. J. Immunol., 1999, 8(6):620-624.

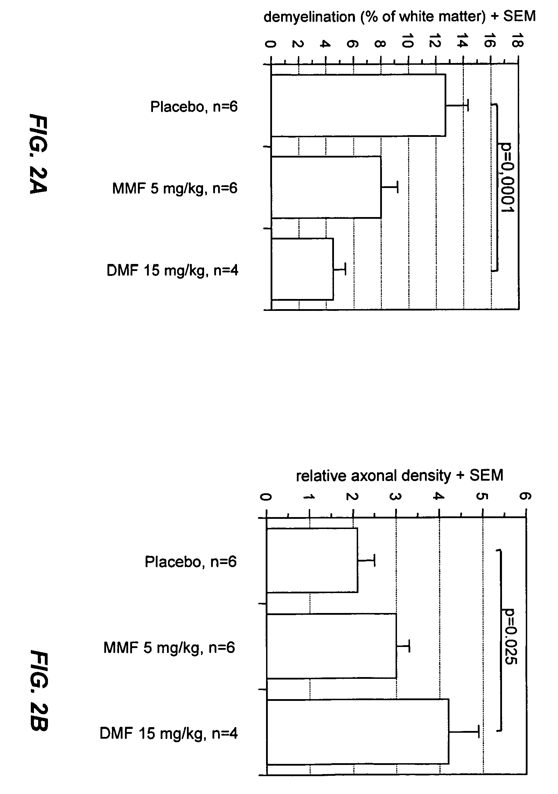

Figure 2A shows the average level of demyelination (% white matter) in a mouse MOG-EAE model 72 days p.i., following administration of DMF or MMF. Demyelination was reduced in the animals treated with DMF and MMF.

Figure 2B shows the level of relative axonal density in a mouse MOG -EAE model 72 days p.i., following administration of DMF or MMF. Axonal loss was reduced in the animals treated with DMF and MMF.

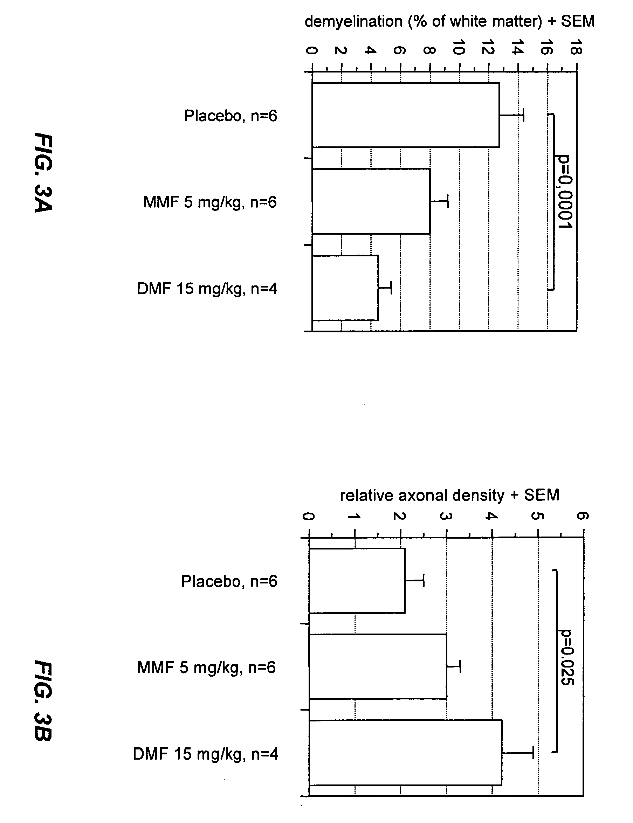

Figure 3A shows results of blinded histological analysis of CD 3 positive T cells infiltrating the spinal cord 72 days after induction of MOG-EAE. Numbers of infiltrating T cells were not significantly different between MMF-treated, DMF-treated or methocel-fed control mice.

Figure 3B shows results of the blinded histological analysis of Mac-3 positive macrophages and microglia infiltrating the spinal cord 72 days after induction of MOG 35-55 EAE. Numbers of infiltrating macrophages and microglia were not significantly different between MMF-treated, DMF-treated, and methocel-fed control mice.

All publications and patent documents cited herein are incorporated by reference in their entirety. To the extent the material incorporated by reference contradicts or is inconsistent with the present specification, the present specification will supersede any such material.

In view of the foregoing, it will be appreciated that the invention described herein inter alia relates to the following items:
1. A method of treating a subject having a neurological disorder characterized by extensive demyelination and axonal loss, the method comprising administering to the subject a therapeutically effective amount of at least one compound of Formula I: 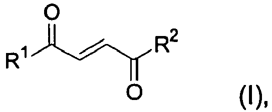 wherein R¹ and R² are independently selected from OH, O⁻, and (C₁₋₆)alkoxy, or a pharmaceutically acceptable salt thereof.
2. The method of item 1, wherein the compound is chosen from dimethyl fumarate and monomethyl fumarate.
3. The method of item 1, wherein the compound is administered in an amount and for a period of time sufficient to reduce demyelination and/or axonal death in the subject.
4. The method of item 1, wherein the compound is administered in an amount and for a period of time sufficient to reduce accumulation of disability in the subject.
6. The method of item 1, wherein the subject has a progressive form of a demyelinating disorder.
7. The method of item 6, wherein the demyelinating disorder is multiple sclerosis.
8. The method of item 7, wherein the subject has secondary progressive multiple sclerosis.
9. The method of item 8, wherein the subject has Devic's disease.
10. The method of item 6, wherein the subject exhibits at least a 1-point increase on the Enhanced Disability Status Scale (EDSS) over a period of one year prior to the administration of the compound.
11. The method of item 6, wherein the subject exhibits at least a 25% increase in T1 lesion load over a period of one year prior to the administration of the compound.
12. The method of item 1, wherein the subject has an EDSS score of at least 3.
13. The method of item 1, wherein the subject has more than 10 hypointense T1 lesions.
14. A method of treating a human subject in need of the treatment, the method comprising treating the subject with a therapeutically effective amount of at least one compound chosen from dimethyl fumarate and monomethyl fumarate, wherein the subject has secondary progressive multiple sclerosis and/or an EDSS score of more than 5.

## Claims

1. At least one compound chosen from dimethyl fumarate and monomethyl fumarate, or a pharmaceutically acceptable salt thereof, for use in treating Acute Disseminated Encephalomyelitis (ADEM) in a subject.

2. The compound or salt for use as in claim 1, wherein the compound is to be administered orally at a therapeutically effective dose.

3. The compound or salt for use as in claim 2, wherein the therapeutically effective dose is administered in separate administrations of 2, 3, 4, or 6 equal doses.

4. The compound or salt for use as in claim 3, wherein the dose is administered in separate administrations of 2 equal doses.

5. The compound or salt for use as in claim 3, wherein the dose is administered in separate administrations of 3 equal doses.

6. The compound or salt for use as in any one of claims 1 to 5, wherein the compound is dimethyl fumarate.

7. The compound or salt for use as in any one of claims 1 to 5, wherein the compound is monomethyl fumarate.

8. The compound or salt for use as in any one of claims 1 to 7, wherein the compound is in the form of pills, tablets, microtablets, pellets, micropellets, capsules, capsules containing microtablets or liquid formulations for oral administration.

9. The compound or salt for use as in any one of claims 1 to 7, wherein the use comprises administering orally one or more capsule(s) containing a pharmaceutical preparation comprising 60 to 240 mg of dimethyl fumarate.

10. The compound or salt for use as in claim 9, wherein the use comprises administering orally one or more capsule(s) containing a pharmaceutical preparation comprising 120 mg of dimethyl fumarate.

11. The compound or salt for use as in claim 9, wherein the use comprises administering orally one or more capsule(s) containing a pharmaceutical preparation comprising 240 mg of dimethyl fumarate.

12. The compound or salt for use as in any one of claims 1 to 11, wherein the compound is administered in the form of a sustained or controlled release formulation.
